# EUROPÄISCHE PATENTSCHRIFT

(11) **EP 2 999 539 B1**
(45) Veröffentlichungstag und Bekanntmachung des Hinweises auf die Patenterteilung: **26.12.2018**
(21) Anmeldenummer: 15736392.0
(22) Anmeldetag: 22.05.2015
(51) Int. Cl.: B01L 9/00

(54) **BEHEIZBARE PINZETTE UND LADEVORRICHTUNG FÜR DIE PINZETTE**
HEATABLE TWEEZERS, AND CHARGING DEVICE FOR THE TWEEZERS
PINCETTE CHAUFFANTE ET CHARGEUR POUR LA PINCETTE

(30) Priorität: 30.05.2014 DE 102014210298
(43) Veröffentlichungstag der Anmeldung: 30.03.2016
(73) Patentinhaber: PFM Medical AG, 50996 Köln (DE)
(72) Erfinder: SCHNEIDER, Erwin, 69226 Nussloch (DE); KRAUS, Steffen, 69207 Sandhausen (DE)
(74) Vertreter: Patent- und Rechtsanwälte Ullrich & Naumann
(86) Internationale Anmeldenummer: PCT/DE2015/200322
(87) Internationale Veröffentlichungsnummer: WO 2015/180722

(56) Entgegenhaltungen:
- WO-A1-2004/008907
- CN-U- 204 255 733
- DE-A1- 4 305 057
- US-A1- 2005 121 495
- US-A1- 2011 046 620
- US-A1- 2011 278 942
- US-B1- 6 235 027

## Beschreibung

Die Erfindung betrifft eine Pinzette, insbesondere zur Handhabung histologischer Präparate, vorzugsweise dünner Gewebeproben, mit zwei an einem Ende elastisch verbundenen Schenkeln und am anderen, freien Ende der Schenkel ausgebildeten Spitzen, die gemeinsam einen Eingriffsbereich bilden, wobei ein mittiger Bereich zum Halten und Betätigen der Schenkel, um nämlich die Spitzen zur gegenseitigen Anlage bzw. Kontaktierung zu bringen, dient.

Unter einer Pinzette ist ganz allgemein ein Werkzeug zu verstehen, welches dazu dient, kleine Gegenstände zu greifen. Meist besteht eine Pinzette aus zwei aneinandergefügten Schenkeln, die aus Metall, Kunststoff, glasfaserverstärktem Kunststoff und Keramik bestehen können. Durch leichten Druck lassen sich die Schenkel bzw. deren Spitzen aufeinander zu bewegen. Bei gegenseitigem Kontakt der endseitigen Spitzen ist die Pinzette geschlossen und greift.

Der Begriff "Spitze" ist im weitesten Sinne zu verstehen. So handelt es sich dabei um das freie Ende der beiden Schenkel, welches dem Wortsinn nach spitz, aber auch rund, mehreckig oder sonst wie geformt sein kann. Die Schenkel der Pinzette können unterschiedlichen Querschnitt aufweisen. Deren Verlauf kann gerade oder gebogen sein. Auch kann die Innenseite der Spitzen gerillt ausgeführt sein, um die Teilchen besser greifen zu können.

Die Histologie (Gewebelehre) befasst sich mit der Untersuchung biologischer Proben und ist als Teilgebiet der Medizin oder Biologie und dabei wiederum als Teilgebiet der Anatomie oder Pathologie zu verstehen. Der Histologe untersucht Gewebeproben, wobei mikrometerdünne, meist gefärbte Gewebeschnitte hergestellt und am Mikroskop untersucht werden. Die feinen, kleinen Gewebsschnitte müssen gehandhabt werden, um sie nämlich zu präparieren, einzubetten und/oder auf einem Objektträger zu positionieren. Zu deren Handhabung werden regelmäßig Pinzetten mit geeignet ausgestalteten Spitzen verwendet.

Um die feinen Gewebeproben nicht zu schädigen, dürfen diese ausschließlich mit geeignet temperierten Werkzeugen/Handhabungsgeräten in Kontakt kommen. Handelt es sich bei dem Handhabungsgerät, wie bspw. bei der Pinzette, um einen metallischen Gegenstand mit hoher Wärmeleitfähigkeit, neigt dieser dazu, sehr schnell die Temperatur einer bspw. kalten Tischoberfläche anzunehmen. Dies ist für die Proben schädlich. Entsprechend behilft man sich bislang dadurch, dass man die Pinzette auf eine Wärmeplatte legt und sich dann der temperierten Pinzette bedient. Dies ist umständlich und garantiert vor allem keine homogene Temperatur der Pinzette. Außerdem kühlt die Pinzette in Ermangelung einer hinreichend großen Masse schnell ab. Eine reproduzierbare Handhabung biologischer Proben ist auf diese Weise nicht möglich.

Aus der US 2011/0046620 A1 ist eine Pinzette zur Handhabung histologischer Präparate sowie eine Ladestation zum Laden und Bereitstellen von Pinzetten bekannt.

Aus der DE 43 05 057 A1 ist eine elektrisch beheizte Pinzette zum Einbetten von Gewebeproben in flüssiges Paraffin bekannt. Die Pinzette arbeitet wie ein Tauchsieder, benötigt nämlich einen elektrischen Anschluss und weist in den Metallspitzen jeweils ein Heizelement auf.

Aus der WO 2004/008907 A1 ist eine Pinzette zur Entfernung von Haaren bekannt. Dabei wird ein Stromschlag auf die Pinzettenspitzen übertragen, um Haarfollikel zu schwächen und daraufhin über einen Ziehmechanismus das geschwächte Haar herauszuziehen.

Aus der 2005/121495 A1 ist eine Vorrichtung zur kabelgebundenen Stromversorgung und Temperaturregelung unterschiedlicher Handgeräte bekannt.

Aus der US 2011/278942 A1 ist eine tragbare Stromversorgung zum Zwecke medizinischer Anwendungen bekannt, wobei die Stromversorgung medizinischer Geräte per Kabel erfolgt.

Aus der US 6,235,027 B1 ist eine tragbare thermische Pinzette für Operationszwecke bekannt.

Der vorliegenden Erfindung liegt die Aufgabe zugrunde, eine Pinzette der gattungsbildenden Art anzugeben, mit der sich Gewebeproben ideal handhaben lassen. Außerdem soll eine Vorrichtung zur Bereitstellung einer solchen Pinzette angegeben werden.

Voranstehende Aufgabe ist durch die Merkmale der nebengeordneten Patentansprüche 1 und 8 gelöst.

Die Spitzen und/oder der Verbindungsbereich der Schenkel ist als Gehäuse mit einem Speichermedium zum Speichern von Energie ausgeführt Das Speichermedium erwärmt unmittelbar oder mittelbar den Eingriffsbereich bzw. die Spitzen auf eine vorgegebene Temperatur.

Die Pinzette wird mit einer vorgegebenen bzw. vorgebbaren - konstanten - Temperatur bereitgestellt. Dazu ist die Pinzette mit einem Speichermedium zum Speichern von Energie ausgestattet, wobei das Speichermedium unmittelbar oder mittelbar den Eingriffsbereich bzw. die Spitzen der beiden Schenkel auf eine vorgegebene Temperatur erwärmt und hält.

Das Speichermedium kann in die Schenkel und/oder den endseitigen Verbindungsbereich der Schenkel integriert sein, wobei es möglich ist, dass gesamte zur Verfügung stehende Volumen auszunutzen. Entsprechend bietet es sich an, die Schenkel und den Verbindungsbereich, d.h. den gesamten Körper der Pinzette, im Sinne eines Gehäuses auszugestalten, wobei sich das Speichermedium im Gehäuse befindet.

Bei dem Speichermedium handelt es sich um einen Wärmespeicher, der durch direkte Wärmekopplung erwärmt wird. Das Gehäuse hat dabei eine wärmeisolierende Eigenschaft, so dass die Wärme nahezu ausschließlich über die Spitzen nach außen abgegeben wird. Entsprechend lässt sich die Wärmespeicherkapazität des Materials bestmöglich nutzen.

Alternativ oder zusätzlich handelt es sich bei dem Speichermedium um einen elektrischen Energiespeicher, wobei dieser eine auf den Eingriffsbereich bzw. die Spitzen wirkende elektrische Heizeinrichtung speist. Bei der Heizeinrichtung handelt es sich in vorteilhafter Weise um eine Widerstandsheizung, die einen wärmeleitenden Kontakt mit dem Eingriffsbereich, d.h. mit den Spitzen, hat.

Die Widerstandsheizung kann ein besonderes Heizelement umfassen, bspw. einen Heizdraht, eine Heizwendel, eine Heizmanschette, eine Heizmatte oder eine Heizpatrone. Endseitig werden die Spitzen in das Heizelement eingesteckt, so dass ein wärmeleitender Kontakt zwischen dem Heizelement und der Spitze hergestellt ist.

Der elektrische Energiespeicher kann als Akkumulator oder als Kondensator bzw. als Anordnung von Kondensatoren ausgeführt sein. Wesentlich dabei ist, dass elektrische Energie gespeichert wird, ähnlich wie bei einer elektrischen Zahnbürste, deren Energiespeicher, im Konkreten dort ein Akkumulator, induktiv beim Einstellen in eine Ladestation geladen wird. Zur Ausgestaltung einer solchen Ladestation werden später die Merkmale der Vorrichtungsansprüche erläutert.

In weiter vorteilhafter Weise ist eine Regelung zur Erwärmung des Eingriffsbereichs auf eine vorgegebene oder vorgebbare bzw. einstellbare Temperatur vorgesehen. Diese Regelung kann eine Art Energiemanagement umfassen, um nämlich mit einem vorgegebenen Energiebetrag eine bestmögliche Temperierung der Pinzette über einen möglichst langen Zeitraum hinweg zu realisieren. Es ist denkbar, dass die Temperatur der Spitzen, in einem gewissen Bereich, einstellbar ist, wobei die Regelung eine optimale Nutzung der zur Verfügung stehenden Energie ermöglicht. Dabei ist wesentlich, dass sich der zu erwärmende Bereich auch tatsächlich nur auf die Spitzen konzentriert, so dass diese mit geringstmöglichem Energiebetrag auf bspw. Körpertemperatur gehalten werden können. Die Spitzen sollten dabei aus einem Metall mit hoher Wärmeleitfähigkeit und großer Wärmespeicherkapazität hergestellt sein. Auch ist es denkbar, dass die Spitzen, ungeachtet des Grundmaterials, mit einem vorzugsweise wärmeleitenden Material beschichtet sind, beispielsweise mit Titan oder einer Titanlegierung. Das Beschichtungsmaterial sollte hart bzw. robust sein, um nämlich den Verschleiß zu reduzieren. So lassen sich die Spitzen robuster gestalten.

Des Weiteren ist es von Vorteil, wenn die Schenkel bzw. deren Gehäuse aus Kunststoff und/oder Keramik gefertigt sind. Wie bereits zuvor erwähnt, sollte das Gehäuse wärmeisolierende Eigenschaften haben. Auf der Außenseite können zur Begünstigung der Haptik des Gehäuses bzw. der Schenkel Maßnahmen wie Noppen, Rillen oder dgl. vorgesehen sein, nämlich Maßnahmen, die die Handhabung begünstigen bzw. erleichtern.

An dieser Stelle sei angemerkt, dass die Pinzette im Rahmen der zuvor genannten Ausgestaltung all diejenigen Elemente beinhalten muss, die zur Energieübertragung und Erwärmung der Spitzen erforderlich sind, ähnlich wie bei einer elektrischen Zahnbürste, dort in Bezug auf die Kinetik. Das Aufladen des Akkus erfolgt regelmäßig induktiv, ebenso wie bei der elektrischen Zahnbürste. Diesbezügliche elektrische/elektronische Elemente und Bausteine können unter Bezugnahme auf ähnlich arbeitende Vorrichtungen von einer weiterreichenden Beschreibung ausgenommen werden.

In erfindungsgemäßer Weise ist zum universellen Einsatz der Pinzette der die Spitze bildende Eingriffsbereich, d.h. die jeweiligen Spitzen, paarweise austauschbar. Die austauschbaren freien Enden der Schenkel, nämlich die Spitzen, können dabei kraft- und/oder formschlüssig, insbesondere über eine Steckverbindung, Rastverbindung, Schraubverbindung oder Bajonettverbindung an die Schenkelstümpfe gekoppelt werden. Ein einfacher Austausch der Spitzen vereinfacht die Handhabung ganz erheblich. So lassen sich bspw. flache "Spitzen" mühelos gegen beliebig geformte, krallenartige Spitzen austauschen, je nach Bedarf. Auch kann die Pinzette durch das Einwechseln geeigneter Spitzen als Schneidgerät/Stanzgerät umfunktioniert werden.

Wie bereits zuvor erwähnt, ist bei einer Ausgestaltung der Pinzette, ähnlich einer elektrischen Zahnbürste, eine elektrische Ladestation vorgesehen, insbesondere zum induktiven Laden des im Körper der Pinzette vorgesehenen Energiespeichers. Dieser kann automatisch eine integrale Heizeinrichtung mit elektrischer Energie versorgen. Auch kann eine externe Stromversorgung die interne bzw. integrale Heizeinrichtung direkt versorgen, nämlich dann, wenn die Pinzette in einer entsprechenden Ladestation steht.

Auch ist es denkbar, dass zur Aktivierung der Heizeinrichtung, bspw. an einem Schenkel der Pinzette, ein Schalter vorgesehen ist, so dass eine Beheizung erst nach Einschalten der Pinzette stattfindet.

Die der Erfindung zugrundeliegende Aufgabe ist in Bezug auf eine Vorrichtung durch die Merkmale des nebengeordneten Anspruchs 8 gelöst. Danach ist eine Vorrichtung zum Bereitstellen mindestens einer gebrauchsfähigen Pinzette entsprechend den voranstehenden Ausführungen gekennzeichnet durch ein eine Station bildendes Gehäuse und einen im Gehäuse, vorzugsweise auf der Oberseite, ausgebildeten Einsteck-/Einstell-/Aufsteckbereich, der zum Positionieren und Laden der Pinzette dient. Die Pinzette wird vorzugsweise stehend, d.h. in vertikaler Positionierung, geladen, ähnlich wie bei einer elektrischen Zahnbürste. Es ist auch denkbar, dass die Pinzette in eine im Gehäuse ausgebildete Lademulde gelegt wird, wobei die induktive Kopplung auch im Rahmen einer solchen Positionierung in bekannter Weise erfolgt.

In Bezug auf ein kontinuierliches Arbeiten mit einer temperierten Pinzette ist es von weiterem Vorteil, wenn das Gehäuse zwei Einsteck-/Einstell-/Aufsteckbereiche aufweist, nämlich für zwei voneinander unabhängige Pinzetten. So kann die eine Pinzette in voll geladenem und beheiztem Zustand verwendet werden, solange, bis die elektrische Energie zum Beheizen der Spitzen bzw. zum Halten auf einem vorgegebenen Temperaturniveau zu Ende geht. Um dies festzustellen, kann die Temperatur in den Spitzen durch einen Temperatursensor ermittelt werden, der über einen entsprechenden Prozessor ein Signal unmittelbar an der Pinzette oder, im Wege einer kontaktlosen Verbindung, über die Station ausgibt. Reicht der Ladezustand des Akkus der Pinzette nicht mehr aus, kann der Benutzer diese in die Ladestation stellen und sich der zweiten Pinzette bedienen. Dabei wird die erste Pinzette geladen. Eine besondere Elektronik zum schnellen Laden kann vorgesehen sein.

Wie bereits zuvor ausgeführt, sind die Einsteck-/Einstell-/Aufsteckbereiche als induktiv arbeitende Ladestationen ausgeführt, wobei die Pinzette ein hermetisches Gehäuse aufweisen kann, wodurch die Verletzungsgefahr aufgrund unsachgerechter Handhabung weitestgehend eliminiert ist. Entsprechend ist das Gehäuse der Station mit einer durchgehenden Oberfläche, ungeachtet der jeweiligen Kontur, ausgestattet, so dass auch von der Station keinerlei Gefahren ausgehen. Außerdem ist die Reinigung der Oberflächen dadurch ganz erheblich vereinfacht.

Die Spitzen der jeweiligen Pinzette sind entsprechend den voranstehenden Ausführungen paarweise austauschbar. Entsprechend weist das Gehäuse erfindungsgemäß mehrere Aufnahmen, insbesondere Einstecköffnungen oder Aufsteckpins, zum Bereitstellen unterschiedlich geformter Spitzenpaare auf. Diese können jeweils nebeneinander stehend bzw. steckend oder liegend vorgesehen sein, und zwar in doppelter Anzahl, sofern die Station zwei Pinzetten umfasst.

Es ist von weiterem Vorteil, wenn die Spitzen/Spitzenpaare im erwärmten und temperierten Zustand bereitgestellt werden. Zum Erwärmen der diesbezüglichen Aufnahmen bzw. der darin befindlichen Spitzenpaare kann eine besondere Heizeinrichtung vorgesehen sein, die entweder als Widerstandsheizung ausgeführt ist oder die unmittelbar induktiv an das Material der Spitzenpaare ankoppelt. Diese Vorkehrung begünstigt den Energiehaushalt des Energiespeichers in der Pinzette, zumal es nicht erforderlich ist, ein eingewechseltes Spitzenpaar von einer niedrigen Bereitstellungstemperatur auf eine bspw. der Körpertemperatur entsprechende Temperatur zu erwärmen.

Gleiches gilt für die Bereitstellung der Pinzette selbst. Sofern sich diese in der Ladestation befindet, kann die Temperierung der Spitzen bereits aktiviert werden, so dass die Pinzetten mit temperierten Spitzen bereitgestellt werden. All diese Funktionen sind über ein vorzugsweise der Anzeige zugeordnetes Bedientableau, vorzugsweise im Rahmen eines Touch-Panels, aktivierbar. Foliengeschützte, in den Bereich des Displays integrierte Schalter können ebenso vorgesehen sein.

Wie bereits erwähnt, umfasst das Gehäuse ein Display, welches vorzugsweise in einem vorderen, insbesondere schräg nach oben geneigten Bereich des Gehäuses integriert ist. Das Display zeigt den Betriebszustand der Station und/oder den Ladezustand der Pinzette an.

In ganz besonders raffinierter Weise kann zwischen der Station und der jeweiligen Pinzette eine drahtlose Verbindung bestehen, vorzugsweis eine Verbindung über Bluetooth, über Hochfrequenzsignale oder aber eine Verbindung über ein WLAN-Netz, wobei im Rahmen einer solchen Vorkehrung auch die Einbindung in eine Computeranlage möglich ist. Auch ist es denkbar, dass die Steuerung der Station über einen Organizer möglich ist, unter Nutzung eines dazu vorgesehenen Programms in Form einer sog. App.

Es ist weiter denkbar, dass über die Station, über das dort vorgesehene Display im Gehäuse, der Lade-/Betriebszustand der Pinzette anzeigbar und ggf. optisch und/oder akustisch dem Benutzer mitteilbar ist. So kann der Benutzer an der Station erkennen, zumindest abschätzen, wie lange er die Pinzette mit hinreichender Temperierung der Spitze noch nutzen kann. Bei Bedarf wird er dann die Pinzette in die Ladestation zurückstellen und sich der zweiten, bevorrateten Pinzette bedienen.

Durch das Zusammenspiel der zuvor erörterten erfindungsgemäßen Vorrichtung und der eingangs erörterten erfindungsgemäßen Pinzette kann das Aufheizen der Pinzette, in der sich eine interne Heizeinrichtung einerseits und ein Akkumulator zum Speichern elektrischer Energie andererseits befindet, wie folgt erfolgen:
Die Pinzette befindet sich in der Ladeschale, d.h. im Einsteck-/Einstell-/Aufsteckbereich und ist dort entsprechend positioniert. In diesem Zustand wird die Vorrichtung, die mindestens eine Pinzette trägt, eingeschaltet. Die Pinzette wird, sofern Sie sich ordnungsgemäß in der Ladestation befindet, direkt mit elektrischer Energie versorgt, wobei die elektrische Energie bzw. der Strom nicht vom Akkumulator der Pinzette kommt. Vielmehr wird die Pinzette, unter Umgehung des Akkumulators bzw. in vorteilhafter Weise bei gleichzeitigem Aufladen des Akkumulators, direkt mit Strom versorgt bzw. wird die interne Heizeinrichtung der Pinzette direkt mit Strom versorgt, so dass die Pinzette, insbesondere die Spitzen der Pinzette auf Betriebstemperatur gebracht werden. Der Ladevorgang des Akkumulators findet gleichzeitig statt.

Wird die Pinzette aus der Ladestation entnommen, wird diese, insbesondere die Spitze der Pinzette, über den internen Akku der Pinzette auf Temperatur gehalten.

Sobald die elektrische Energie des internen Akkumulators, beispielsweise über eine Zeitdauer von 30 Minuten hinweg, aufgebraucht ist, ertönt ein akustisches Signal oder wird ein optisches Signal, beispielsweise eine Warnleute, ausgelöst, wodurch dem Benutzer mitgeteilt wird, dass die Pinzette wieder in die Ladestation der Vorrichtung abgelegt werden muss, so dass der Akku erneut geladen werden kann. Befindet sich die Pinzette wieder in der Ladestation, wiederholt sich der Vorgang entsprechend den voranstehenden Ausführungen, wird nämlich die interne Heizeinrichtung direkt mit Strom versorgt und werden die Pinzettenspitzen beheizt, während der Akkumulator geladen wird.

Bei Vorkehrung zweier Ladestationen und entsprechend mindestens zweier Pinzetten steht die zweite Pinzette "geladen" zur Verfügung, d.h. die Spitzen der Pinzette befinden sich auf Betriebstemperatur und der Akkumulator der zweiten Pinzette ist "voll". Diese zweite Pinzette kann dann genutzt werden, während zu der ersten Pinzette der Ladevorgang - Beheizen der Pinzettenspitzen durch direkte Stromversorgung und Aufladen des Akkumulators - erfolgt.

Während des Ladevorgangs übernimmt die Vorrichtung bzw. die Ladestation die Energiezufuhr, bis die gewünschte Temperatur in der Pinzettenspitze erreicht ist. Bei Bedarf kann die Pinzette dann wieder getauscht werden, so dass ein kontinuierliches Arbeiten bei Vorkehrung mindestens zweier Pinzetten möglich ist.

Grundsätzlich ist es auch denkbar, dass der Pinzette bzw. den Pinzettenspitzen die Temperatur unmittelbar über die Ladestation vermittelt wird, ohne dass in der Pinzette eine interne Heizeinrichtung vorgesehen ist. Insoweit würde die Pinzette, bei Vorkehrung eines entsprechenden Wärmespeichers, thermisch geladen werden und könnte die Pinzette so lange genutzt werden, solange der Wärmespeicher die Pinzettenspitze hinreichend mit Temperatur versorgt, so dass die Pinzettenspitzen auf Betriebstemperatur gehalten werden. Bei Unterschreiten der Betriebstemperatur könnte ein Warnsignal abgesetzt werden, so dass die erste Pinzette, bei nicht mehr hinreichend "gefülltem" Wärmespeicher, gegen die zweite, thermisch geladene Pinzette ausgetauscht wird.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht ein Ausführungsbeispiel einer Vorrichtung zum Bereitstellen von zwei gebrauchsfertigen Pinzetten mit austauschbaren Spitzen, wobei das Gehäuse der Vorrichtung ein Display zum Anzeigen des Betriebszustands der Station und des Ladezustands der Pinzetten aufweist,
- Fig. 2: in einer schematischen Ansicht, schräg von hinten, den Gegenstand aus Figur 1,
- Fig. 3: in einer schematischen Ansicht, von vorne, den Gegenstand aus Figur 1,
- Fig. 4: in einer schematischen Ansicht, von hinten, den Gegenstand aus Figur 1
- Fig. 5: in einer ersten schematischen Seitenansicht den Gegenstand aus Figur 1,
- Fig. 6: in einer zweiten schematischen Seitenansicht, den Gegenstand aus Figur 1 von der anderen Seite,
- Fig. 7: in einer schematischen Draufsicht den Gegenstand aus Figur 1 von oben,und
- Fig. 8: in einer schematischen Ansicht, von unten, den Gegenstand aus Figur 1.

Die Figuren zeigen ein Ausführungsbeispiel einer erfindungsgemäßen Vorrichtung zum Bereitstellen zweier erfindungsgemäßer Pinzetten 1 und zum Austausch gedachter Spitzen 2, die unterschiedlich geformt sind. Die Spitzen 2 werden paarweise bevorratet bzw. zum Austausch zur Verfügung gestellt.

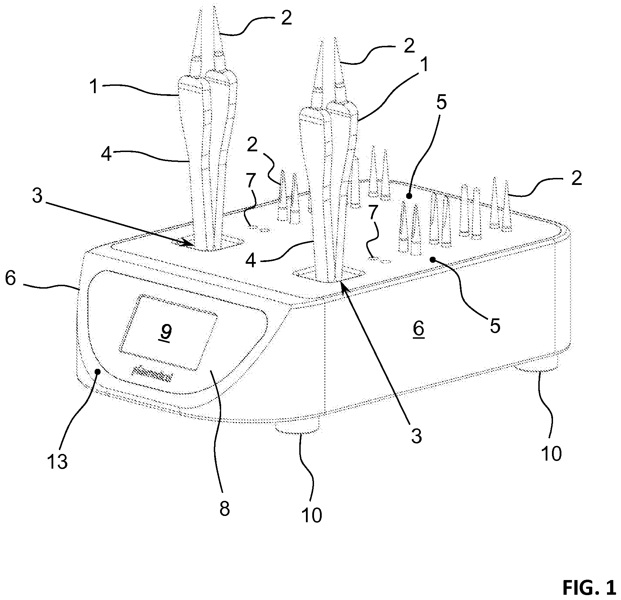

Fig. 1 zeigt die Vorrichtung in einer schematischen Ansicht, wobei dort zwei Pinzetten 1 jeweils in einer Ladestation 3 stehen. Die Pinzetten 1 sind ähnlich einer elektrischen Zahnbürste mit einem induktiv aufladbaren Akkumulator ausgestattet, der eine in der Pinzette 1 integrierte Heizeinrichtung mit elektrischer Energie speist. Nicht gezeigte Heizelemente dienen zur Temperierung der Spitzen 2, die endseitig in die Schenkel 4 der Pinzette in temperierte Aufnahmen einsteckbar oder auf temperierte Halteelemente aufsteckbar sind.
Fig. 1 zeigt deutlich, dass die Ladestation 3 in der Oberfläche 5 des Gehäuses eingearbeitet ist, so dass sich die Pinzette 1 sicher in die Ladestation 3 einstecken bzw. einstellen lässt. Ein sicherer Halt der Pinzette 1 ist gewährleistet.

Fig. 1 zeigt des Weiteren, dass sich in der Oberfläche 5 des Gehäuses 6 weitere Ausnehmungen 7 befinden, die zum paarweisen Einstecken von Spitzen 2 dienen. So lassen sich Spitzen 2 mit unterschiedlicher Gestalt bevorraten, mit denen sich die Pinzette 1 - im Austausch - bestücken lässt. Eine Beheizung der Spitzen 2 entsprechend der allgemeinen Beschreibung ist vorgesehen.

Das Gehäuse 6 ist geprägt durch eine harmonische, abgerundete Form und durch eine vorderseitig vorgesehene Bedienfläche 8, die schräg nach oben geneigt ist, um dem Benutzer die Handhabung zu erleichtern. In die Bedienfläche 8 ist ein Display 9 integriert, welches als Touch-Panel ausgestattet sein kann. Im Rahmen einer solchen Vorkehrung sind keinerlei in das Gehäuse 6 führende Kanten/Stoßstellen, etc. vorgesehen, so dass das Gehäuse 6 hermetisch dicht ausgeführt sein kann. Das Gehäuse 6 kann aus Aluminium oder aus Kunststoff bestehen, vorzugsweise identisch auf allen Seiten, wobei eine unterschiedliche Farbgebung und/oder Oberflächenbeschichtung möglich ist.

Fig. 1 zeigt des Weiteren, dass das Gehäuse 6 auf insgesamt vier Gummifüßen 10 steht, die dem Gehäuse 6 einen zum Untergrund beabstandeten, sicheren Stand ermöglichen.

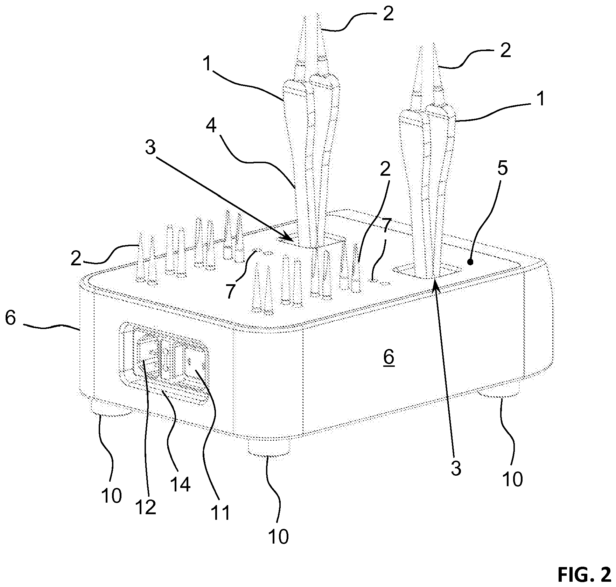

Fig. 2 zeigt die Vorrichtung aus Fig. 1 in einer schematischen Ansicht, schräg von hinten, so dass sich in Verbindung mit der Darstellung nach Fig. 1 ein Gesamteindruck ergibt. Rückseitig ist ein Ein-/Ausschalter 11 vorgesehen, der vertieft im Gehäuse sitzt, so dass ein unbeabsichtigtes Ein- oder Ausschalten weitestgehend verhindert ist. Außerdem ist eine Anschlussbuchse 12 zum Einstecken eines Gerätesteckers für einen Starkstromanschluss vorgesehen.

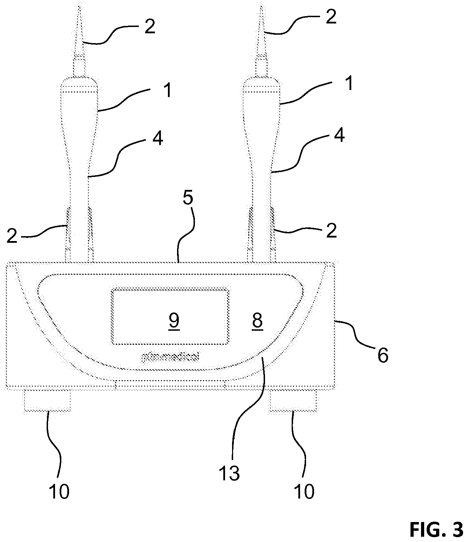

Fig. 3 zeigt den Gegenstand aus Fig. 1 in einer Vorderansicht, die die Form der Pinzette 1 im Bereich der Schenkel 4 zeigt. Das Display 9 und die zur Integration des Displays 9 dienende Oberfläche 13 ist harmonisch angepasst, kann jedoch unterschiedlich designt sein.

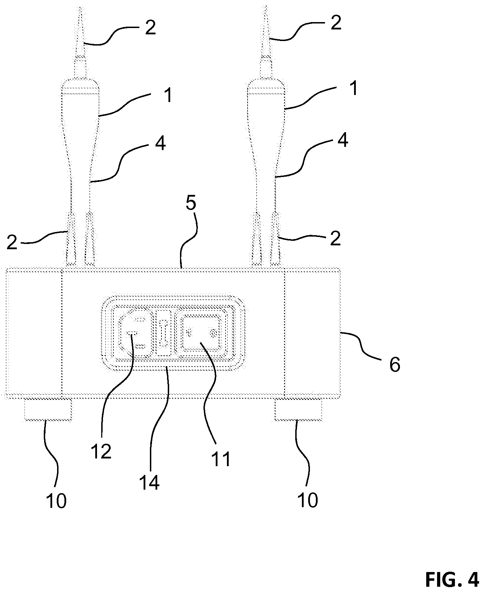

Fig. 4 zeigt die Vorrichtung aus Fig. 1 in einer Ansicht von der Rückseite, mit dem Ein-/Ausschalter 11 und der Anschlussbuchse 12. Beide sind in einen Schalterrahmen 14 integriert.

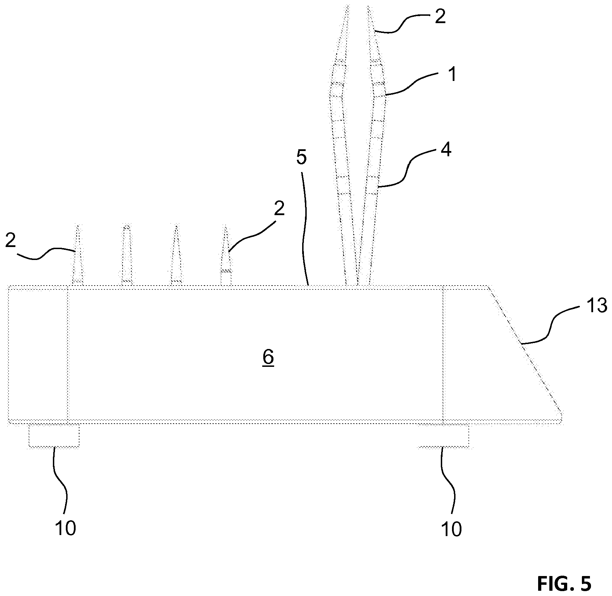

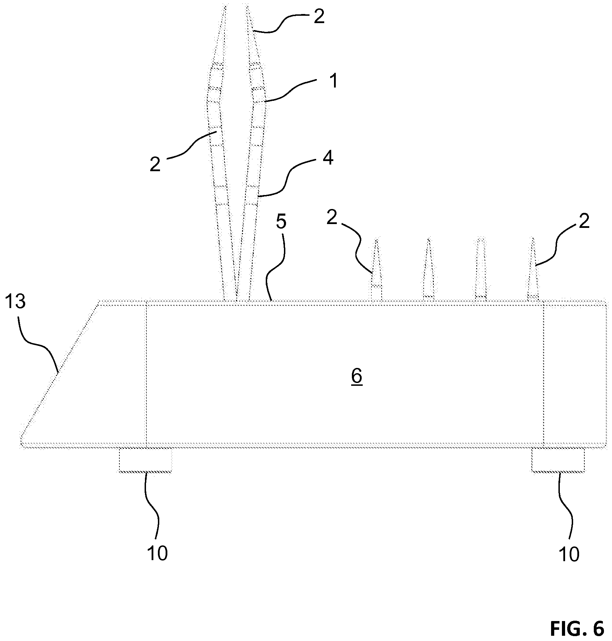

Die Fig. 5 und 6 zeigen die Vorrichtung aus Fig. 1 von der Seite, wonach klar wird, dass das Gehäuse 6 keinerlei störende Elemente in der Seitenwandung hat. Ganz im Gegenteil ist das Gehäuse 6 glatt mit einer einheitlichen Oberfläche ausgeführt. Die Fig. 5 und 6 zeigen die dort zum Aufladen eingestellte Pinzette 1 und den Verlauf der Schenkel 4 von der Seite, mit eingesteckten Spitzen 2. Auch sind die bevorrateten Spitzen 2 gut zu erkennen.

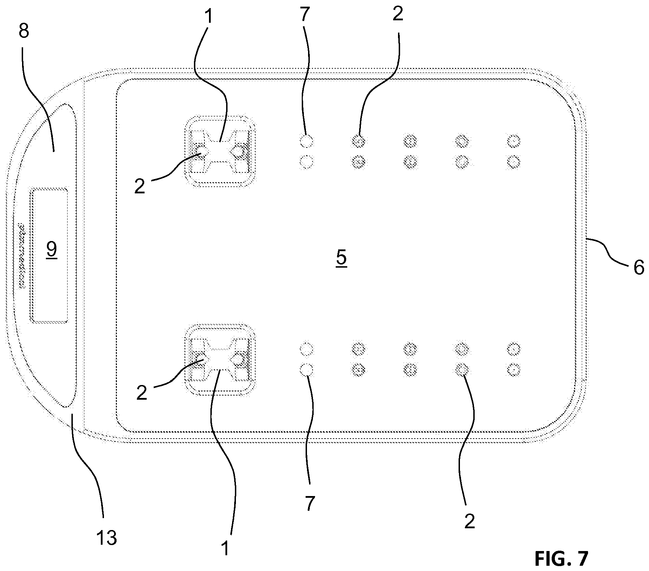

Fig. 7 zeigt den Gegenstand aus Fig. 1 in einer Draufsicht, die die Oberfläche 5 zum Einstellen der Pinzetten 1 und Spitzen 2 und die Oberfläche 13 mit integriertem Display 9 erkennen lässt.

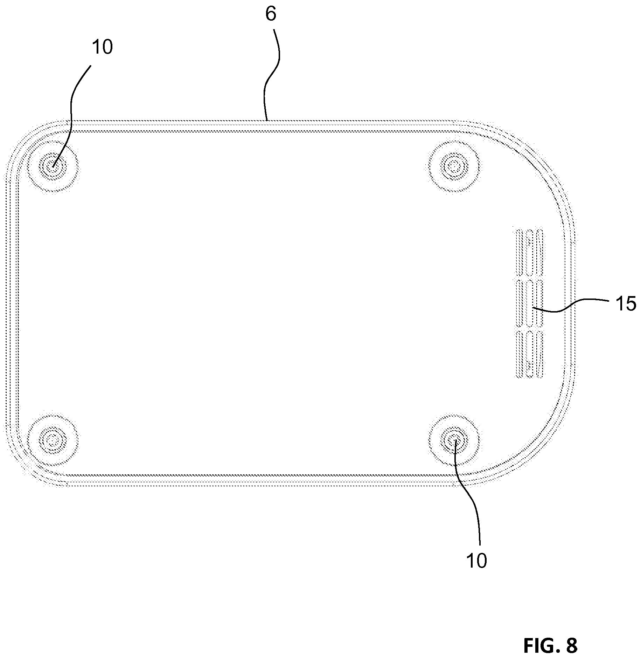

Schließlich zeigt Fig. 8 den Gegenstand aus Fig. 1 in einer Ansicht von unten. Die Gummifüße 10 zur sicheren Positionierung der Vorrichtung sind erkennbar. Außerdem sind Lüftungsschlitze 15 vorgesehen, wobei diese nicht zwingend erforderlich sind. Es ist denkbar, im Inneren des Gehäuses 6 entstehende Wärme über das Gehäuse abzuführen, sofern eine hinreichende Wärmeleitfähigkeit des Gehäuses 6 besteht.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Patentansprüche verwiesen.

Schließlich sei ausdrücklich darauf hingewiesen, dass das voranstehend beschriebene Ausführungsbeispiele der erfindungsgemäßen Vorrichtung lediglich zur Erörterung der beanspruchten Lehre dient, diese jedoch nicht auf das Ausführungsbeispiel einschränkt.

### Bezugszeichenliste

- 1: Pinzette
- 2: Spitze
- 3: Ladestation
- 4: Schenkel (der Pinzette)
- 5: Oberfläche (zum Einstellen der Pinzette)
- 6: Gehäuse (Einstecköffnung)
- 7: Ausnehmung (in der Oberfläche 5)
- 8: Bedienfläche
- 9: Display (in der Bedienfläche)
- 10: Gummifuß
- 11: Ein-/Ausschalter
- 12: Anschlussbuchse
- 13: Oberfläche (für Display)
- 14: Schalterrahmen
- 15: Lüftungsschlitze

## Patentansprüche

1. Pinzette (1) zur Handhabung histologischer Präparate, vorzugsweise dünner Gewebeproben, mit zwei an einem Ende elastisch verbundenen Schenkeln (4) und am anderen, freien Ende der Schenkel (4) ausgebildeten Spitzen (2), die gemeinsam einen Eingriffsbereich bilden, wobei ein mittiger Bereich zum Halten und Betätigen der Schenkel (4), um nämlich die Spitzen (2) zur gegenseitigen Kontaktierung zu bringen, dient, dass die Spitzen (2) und/oder der Verbindungsbereich der Schenkel (4) als Gehäuse mit einem Speichermedium zum Speichern von Energie ausgeführt ist und dass das Speichermedium unmittelbar oder mittelbar den Eingriffsbereich bzw. die Spitzen (2) auf eine vorgegebene Temperatur erwärmt, wobei das Speichermedium ein Wärmespeicher ist und das Gehäuse wärmeisolierende Eigenschaft hat und/oder ein elektrischer Energiespeicher ist und dieser eine auf den Eingriffsbereich wirkende elektrische Heizeinrichtung speist, die einen wärmeleitenden Kontakt mit dem Eingriffsbereich hat, wobei ein Einsteck-/Einstell-/Aufsteckbereich als Ladestation (3) zum Bereitstellen und Positionieren der Pinzette (1) und thermischen und/oder elektrischen Laden, insbesondere zum induktiven Laden des Energiespeichers und/oder zur direkten Stromversorgung einer internen Heizeinrichtung, vorgesehen ist, wobei das thermische und/oder elektrischen Laden, insbesondere das induktive Laden des Energiespeichers und/oder die direkte Stromversorgung einer internen Heizeinrichtung, dann erfolgt, wenn die Pinzette (1) in einer entsprechenden Ladestation (3) positioniert ist,
**dadurch gekennzeichnet, dass** der die Spitze (2) bildende Eingriffsbereich durch unterschiedlich geformte Spitzenpaare austauschbar ist.

2. Pinzette nach Anspruch 1, **dadurch gekennzeichnet, dass** die elektrische Heizeinrichtung eine Widerstandsheizung ist, wobei die Widerstandsheizung als Heizelement einen Heizdraht, eine Heizwendel, eine Heizmanschette, eine Heizmatte oder eine Heizpatrone aufweisen kann.

3. Pinzette nach Anspruch 2, **dadurch gekennzeichnet, dass** der elektrische Energiespeicher als Akkumulator oder als Kondensator ausgeführt ist.

4. Pinzette nach einem der Ansprüche 1 bis 3, **dadurch gekennzeichnet, dass** eine Regelung zur Erwärmung des Eingriffsbereichs auf eine vorgegebene oder vorgebbare bzw. einstellbare Temperatur vorgesehen ist.

5. Pinzette nach einem der Ansprüche 1 bis 4, **dadurch gekennzeichnet, dass** die Spitzen (2) aus Metall gefertigt sind, wobei die Spitzen, ungeachtet des Grundmaterials, mit wärmeleitendem und/oder hartem bzw. robustem Material beschichtet sein können, beispielsweise mit Titan oder einer Titanlegierung.

6. Pinzette nach einem der Ansprüche 1 bis 5, **dadurch gekennzeichnet, dass** die Schenkel (4) und/oder das Gehäuse aus Kunststoff und/oder aus Keramik gefertigt ist/sind.

7. Pinzette nach einem der Ansprüche 1 bis 6, **dadurch gekennzeichnet, dass** die austauschbaren freien Enden der Schenkel (4), d.h. die Spitzen (2), kraft-und/oder formschlüssig, insbesondere über eine Steckverbindung, Rastverbindung, Schraubverbindung oder Bajonettverbindung, mit den Schenkeln (4) verbindbar ist.

8. Vorrichtung zum Bereitstellen mindestens einer gebrauchsfertigen Pinzette (1) nach einem der Ansprüche 1 bis 7, mit einem eine Station bildenden Gehäuse (6) und einem im Gehäuse (6), auf bzw. in der Oberseite (5), als Ladestation(en) ausgebildeten Einsteck-/Einstell-/Aufsteckbereich zum vorzugsweise vertikalen Positionieren der mindestens einen Pinzette (1) und zum thermischen und/oder elektrischen Laden des Energiespeichers und/oder zur direkten Stromversorgung einer internen Heizeinrichtung, dann, wenn die Pinzette (1) in einer entsprechenden Ladestation (3) positioniert ist, wobei das Gehäuse (6) mehrere Aufnahmen, insbesondere Einstecköffnungen (7) oder Aufsteckpins, zum Bereitstellen unterschiedlich geformter Spitzenpaare hat und zwei Einsteck-/Einstell-/Aufsteckbereiche für zwei Pinzetten (1) haben kann.

9. Vorrichtung nach Anspruch 8, **dadurch gekennzeichnet, dass** der/die Einsteck-/Einstell-/Aufsteckbereich(e) als induktiv arbeitende Ladestatione(n) (3) ausgeführt ist/sind.

10. Vorrichtung nach Anspruch 9, **dadurch gekennzeichnet, dass** zum Erwärmen der Aufnahmen bzw. der darin befindlichen Spitzenpaare eine Heizeinrichtung vorgesehen ist, die als Widerstandsheizung ausgeführt ist oder unmittelbar induktiv an die Spitzenpaare ankoppelt.

11. Vorrichtung nach einem der Ansprüche 8 bis 10, **dadurch gekennzeichnet, dass** das Gehäuse (6), vorzugsweise in einem vorderen, insbesondere schräg gestellten Bereich, ein Display (9) umfasst, welches den Betriebszustand der Station (3) und/oder den Ladezustand der Pinzette(n) (1) zeigt.

12. Vorrichtung nach einem der Ansprüche 8 bis 11, **dadurch gekennzeichnet, dass** zwischen der Station (3) und der Pinzette eine drahtlose Verbindung besteht, vorzugsweise über Bluetooth, über die der Lade-/Betriebszustand der Pinzette (1) an die Station (3) übermittelt und ggf. optisch und/oder akustisch angezeigt wird.

## Claims

1. Pair of tweezers (1) for handling histological preparations, preferably thin tissue samples, having two members (4) which are resiliently connected at one end and tips (2) which are formed at the other, free end of the members (4), which together form an engagement region, wherein a central region serves to retain and activate the members (4) in order to bring the tips (2) into mutual contact, in that the tips (2) and/or the connection region of the members (4) is constructed as a housing with a storage medium for storing energy, and in that the storage medium heats the engagement region or the tips (2) directly or indirectly to a predetermined temperature, wherein the storage medium is a heat store and the housing has a thermally insulating property and/or is an electrical energy store and it supplies an electric heating device which acts on the engagement region and which has a thermally conductive contact with the engagement region, wherein an insertion/adjustment/fitting region is provided as a loading station (3) for providing and positioning the pair of tweezers (1) and thermally and/or electrically charging, in particular for inductively charging, the energy store and/or for direct power supply of an internal heating device, wherein the thermal and/or electrical charging, in particular the inductive charging of the energy store and/or the direct power supply of an internal heating device, is carried out when the pair of tweezers (1) is positioned in a corresponding charging station (3),
**characterised in that** the engagement region which forms the tip (2) can be replaced by differently shaped tip pairs.

2. Pair of tweezers according to claim 1, **characterised in that** the electric heating device is a resistance heater, wherein the resistance heater may have as a heating element a heating wire, a heating coil, a heating sleeve, a heating mat or a heating cartridge.

3. Pair of tweezers according to claim 2, **characterised in that** the electric energy store is constructed as an accumulator or as a capacitor.

4. Pair of tweezers according to any one of claims 1 to 3, **characterised in that** a control for heating the engagement region to a predetermined or predeterminable or adjustable temperature is provided.

5. Pair of tweezers according to any one of claims 1 to 4, **characterised in that** the tips (2) are produced from metal, wherein the tips, regardless of the base material, may be coated with thermally conductive and/or hard or robust material, for example, with titanium or a titanium alloy.

6. Pair of tweezers according to any one of claims 1 to 5, **characterised in** the members (4) and/or the housing is/are produced from plastics material and/or from ceramic material.

7. Pair of tweezers according to any one of claims 1 to 6, **characterised in that** the replaceable free ends of the members (4), that is to say, the tips (2), can be connected to the members (4) in a non-positive-locking and/or positive-locking manner, in particular by means of a plug type connection, locking connection, screw connection or bayonet connection.

8. Device for providing at least one ready-to-use pair of tweezers (1) according to any one of claims 1 to 7, having a housing (6) which forms a station and an insertion/adjustment/fitting region which is constructed in the housing (6) on or in the upper side (5) as a charging station(s) for preferably vertically positioning the at least one pair of tweezers (1) and for thermally and/or electrically charging the energy store and/or for the direct power supply of an internal heating device at times when the pair of tweezers (1) is positioned in a corresponding charging station (3), wherein the housing (6) has a plurality of receiving members, in particular insertion openings (7) or insertion pins, for providing differently shaped tip pairs and may have two insertion/adjustment/fitting regions for two pair of tweezers (1).

9. Device according to claim 8, **characterised in that** the insertion/adjustment/fitting region(s) is/are constructed as inductively operating charging station(s) (3).

10. Device according to claim 9, **characterised in that**, in order to heat the receiving members or the tip pairs which are located therein, there is provided a heating device which is constructed as a resistance heater or which couples directly in an inductive manner to the tip pairs.

11. Device according to any one of claims 8 to 10, **characterised in that** the housing (6) comprises, preferably in a front, in particular obliquely positioned region, a display (9) which shows the operating state of the station (3) and/or the charging state of the pair of tweezers (1).

12. Device according to any one of claims 8 to 11, **characterised in that** between the station (3) and the pair of tweezers there is a wireless connection, preferably via Bluetooth, via which the charging/operating state of the pair of tweezers (1) is transmitted to the station (3) and where applicable is indicated optically and/or acoustically.

## Revendications

1. Pincette (1) destinée à la manipulation de préparations histologiques, de préférence de minces échantillons tissulaires, avec deux branches (4) raccordées élastiquement au niveau d'une extrémité, et des pointes (2), constituées au niveau de l'autre extrémité libre des branches (4), qui forment conjointement une zone de mise en prise, une zone centrale servant à retenir et actionner les branches (4) afin d'amener en fait les pointes (2) à entrer en contact l'une avec l'autre, les pointes (2) et/ou la zone de raccordement des branches (4) étant réalisée(s) en tant que boîtier avec un support de stockage destiné au stockage d'énergie, et le support de stockage chauffant directement ou indirectement la zone de mise en prise ou respectivement les pointes (2) à une température prédéfinie, le support de stockage étant une réserve de chaleur, et le boîtier ayant une caractéristique d'isolation thermique, et/ou étant une réserve d'énergie électrique, et celle-ci alimentant un système de chauffage électrique qui agit sur la zone de mise en prise et qui a un contact thermiquement conducteur avec la zone de mise en prise, une zone d'insertion/de réglage/d'embrochement étant prévue en tant que station de charge (3) pour la fourniture et le positionnement de la pincette (1) et pour le chargement thermique et/ou électrique, en particulier pour le chargement inductif de la réserve d'énergie et/ou pour l'alimentation directe en courant d'un système de chauffage interne, le chargement thermique et/ou électrique, en particulier le chargement inductif de la réserve d'énergie et/ou l'alimentation directe en courant d'un système de chauffage interne s'effectuant quand la pincette (1) est positionnée dans une station de charge (3) correspondante,
**caractérisée en ce que** la zone de mise en prise formant la pointe (2) peut être remplacée par des paires de pointe de formes différentes.

2. Pincette selon la revendication 1, **caractérisée en ce que** le système de chauffage électrique est un chauffage par résistance, le chauffage par résistance pouvant comporter en tant qu'élément chauffant un fil chauffant, un serpentin chauffant, une manchette chauffante, un tapis chauffant ou une cartouche chauffante.

3. Pincette selon la revendication 2, **caractérisée en ce que** la réserve d'énergie électrique est réalisée en tant qu'accumulateur ou en tant que condensateur.

4. Pincette selon l'une des revendications 1 à 3, **caractérisée en ce qu'**il est prévu une régulation destinée au chauffage de la zone de mise en prise à une température prédéfinie ou pouvant être prédéfinie ou réglable.

5. Pincette selon l'une des revendications 1 à 4, **caractérisée en ce que** les pointes (2) sont fabriquées en métal, les pointes pouvant, indépendamment du matériau de base, être revêtues de matériau thermiquement conducteur et/ou de matériau dur et/ou robuste, par exemple de titane ou d'un alliage de titane.

6. Pincette selon l'une des revendications 1 à 5, **caractérisée en ce que** les branches (4) et/ou le boîtier est/sont fabriqué(es) en matière plastique et/ou en céramique.

7. Pincette selon l'une des revendications 1 à 6, **caractérisée en ce que** les extrémités libres remplaçables des branches (4), c'est-à-dire les pointes (2), peuvent être raccordées aux branches (4) par liaison de force et/ou de forme, en particulier par le biais d'un raccordement enfichable, d'un raccordement par encliquetage, d'un raccordement vissé ou d'un raccordement à baïonnette.

8. Dispositif destiné à la fourniture d'au moins une pincette (1) prête à l'emploi selon l'une des revendications 1 à 7, avec un boîtier (6) formant une station et avec une zone d'insertion/de réglage/d'embrochement constituée en tant que station(s) de charge dans le boîtier (6), sur ou respectivement dans le côté supérieur (5), pour le positionnement de préférence vertical de la pincette (1) au moins au nombre de un et pour le chargement thermique et/ou électrique de la réserve d'énergie et/ou pour l'alimentation directe en courant d'un système de chauffage interne quand la pincette (1) est positionnée dans une station de charge (3) correspondante, le boîtier (6) ayant plusieurs logements, en particulier des ouvertures d'enfichage (7), ou des goupilles d'embrochement, pour la fourniture de paires de pointes de formes différentes, et pouvant avoir deux zones d'insertion/de réglage/d'embrochement pour deux pincettes (1).

9. Dispositif selon la revendication 8, **caractérisé en ce que** la/les zone(s) d'insertion/de réglage/d'embrochement est/sont réalisée(s) en tant que station(s) de charge (3) fonctionnant de façon inductive.

10. Dispositif selon la revendication 9, **caractérisé en ce que**, pour le chauffage des logements ou respectivement des paires de pointes qui s'y trouvent, il est prévu un système de chauffage qui est réalisé en tant que chauffage par résistance ou qui est couplé directement par induction aux paires de pointes.

11. Dispositif selon l'une des revendications 8 à 10, **caractérisé en ce que**, de préférence dans une zone avant, en particulier placée de façon oblique, le boîtier (6) comprend un affichage (9) qui montre l'état de fonctionnement de la station (3) et/ou l'état de charge de la/des pincette(s) (1).

12. Dispositif selon l'une des revendications 8 à 11, **caractérisé en ce que**, entre la station (3) et la pincette, il y a une liaison sans fil, de préférence via Bluetooth, par le biais de laquelle l'état de charge/de fonctionnement de la pincette (1) est transmis à la station (3) et est éventuellement affiché optiquement et/ou acoustiquement.
